# EUROPEAN PATENT SPECIFICATION

(11) **EP 0 971 707 B1**
(45) Date of publication and mention of the grant of the patent: **30.10.2002**
(21) Application number: 97914499.5
(22) Date of filing: 04.04.1997
(51) Int. Cl.: A61K 31/18

(54) **COMPOSITIONS FOR THE TREATMENT OF BURNS AND EXTERNAL ULCERS AND A PROCESS FOR THE PREPARATION THEREOF**
ZUBEREITUNGEN ZUR BEHANDLUNG VON VERBRENNUNGEN UND EXTERNEN ULCERA SOWIE VERFAHREN ZU DEREN HERSTELLUNG
COMPOSITIONS DE TRAITEMENT DE BRULURES ET DE PLAIES EXTERNES ET PROCEDE DE PREPARATION ASSOCIE

(43) Date of publication of application: 19.01.2000
(73) Proprietor: Human Oltoanyagtermelö és Gyogyszergyarto Rt., 2100 Gödöllö (HU)
(72) Inventor: Lukacs, Karoly, 7400 Kaposvar (HU)
(74) Representative: Schwarz, Albin, Dr.
(86) International application number: HU9700014
(87) International publication number: WO98044914

(56) References cited:
- EP-A- 0 343 671
- WO-A-91/12008
- WO-A-91/17754
- RO-A- 83 134
- RO-A- 83 135
- US-A- 4 535 078
- US-A- 4 777 171
- "ROTE LISTE" 1995 , ECV EDITIO CANTOR , AULENDORF/WÜRTT. GERMANY XP002052848 No.:84038 "Babix-Wundsalbe N" Nos.:31376, 31381, 84032-84038
- DATABASE WPI Section Ch, Week 9344 Derwent Publications Ltd., London, GB; Class D21, AN 93-347307 XP002052849 & HU 63 552 A (GAL C) , 28 September 1993
- DATABASE WPI Section Ch, Week 7950 Derwent Publications Ltd., London, GB; Class B05, AN 79-90053B XP002052850 & JP 54 140 712 A (YAGIMOTO Y) , 1 November 1979

## Description

### Field of the invention

The invention relates to compositions for the treatment of bums and external ulcers and a process for the preparation thereof.

### Background of the invention

There are millions of burnings occurring all over the world every year. The treatment thereof depends on the expansion and the depth of the wound, the age of the patient and the additional diseases or injuries. The initial attention focuses on the cooling and the fluid intake. The next step is the prevention of the infection, followed by the protection of the spontaneously epithelising burn against desiccation and further injury. Areas incapable of spontaneous epithelisation should be excised and skin transplantation should be carried out (Modern Medical Diagnostics and Therapy, edited by: Schroder, Steven A.; Krupp, Marcus A.; Tierney, Lawrence M. Jr., and MCPnee, Stephen J.; Officina Nova Kiadó (Publishing House), 1990., pp.1160-1166).

Infection can be avoided by the application of antibiotics. Antibiotics penetrating entirely into the wound, being effective against both Gram negative and Gram positive bacteria as well as Candida, not inflicting pain and having no side effects, are, however, presently non-existent. Silver sulfadiazine is widely used, though its disadvantages lie in the fact that it is not effective against all Pseudomonas strains, it may cause leucopenia and fever and also might delay epithelisation. Mafenide is more effective against Pseudomonas, though it delays epithelisation and may provoke pain. Povidone-iodine is especially effective against Candida, Gram negative or Gram positive pathogens but hardly penetrates the scab, dehydrates the wound area and its use is painful.

Pain is a significant factor in the first phase of healing, that is when the wound closes, so the application of painkillers is necessary but special care is to be taken in determining the sufficient dose.

There are several burn treating sprays and ointments, e. g. Panthenol spray, Phlogosam ointment or Irix spray available in pharmacies. Their common disadvantage is that additional pain alleviation is indispensable, they are unsuitable for treating expansive injuries, and they do not provide any protection against bacteria.

The optimal way of healing a wound would be if the bum was healed from the inside out and then epithelised possibly without pain or scarring. The aim of the invention is to provide such a product.

### Disclosure of the invention

The invention is based on the recognition that if a sulfonamide is combined with a macrolide antibiotic or with chloramphenicol or thiamphenicol, as well as with zinc oxide, camomile oil and azulene, the mixture thus obtained will cure burns from the inside out, continuously epithelising them in the way that they do not develop a "scarline" around the wound and the process takes place without pain and the risk of infection is reduced to a minimum. An outstanding result is achieved if the mixture contains more than one different sulfonamide.

As sulfonamide preferably sulfadimidine or sulfamethoxazole can be used.

The requirements macrolide antibiotics should meet are as follows: bad or no solubility in water, they should be effective also in the case of lacerated tissues, should not hinder cell-division, should not cause cell proliferation and they should be chemically stable i.e. they could not be degraded by the body fluids. As a macrolide antibiotic preferably primycin-sulfate is used.

The invention relates to a composition for treating burns and external ulcers which contains as active ingredient the mixture of one or more sulfonamides, especially sulfadimidine, a macrolide antibiotic, especially primycin-sulfate or chloramphenicol or thiamphenicol, as well as zinc oxide, camomile oil and azulene. The weight ratio of the sulfonamide, the macrolide antibiotic, zinc oxide, camomile oil and azulene is preferably 0.1-15.0 : 0.1-1.0 : 0.1-25.0 : 0.3-5.0 : 0.01-1.0, especially 2.0 -10.0 : 0.5-2.0 : 1.5-8.3 : 0.3-3.28 : 0.01-0.12. If instead of the macrolide antibiotic chloramphenicol or thiamphenicol is used in the above mixture, its preferable weight ratio is 0.01-7.0.

The invention also relates to a process for the preparation of compositions for the treatment of burns and external ulcers by mixing one or more sulfonamides, especially sulfadimidine with a macrolide antibiotic, especially primycin-sulfate, zinc oxide, camomile oil and azulene, preferably in the weight ratio of 0.1-15.0 : 0.1-1.0 : 0.1-25.0 : 0.3-5.0 : 0.01-1.0, especially in the weight ratio of 2.0-10.0 : 0.5-2.0 : 1.5-8.3 : 0.3-3.28 : 0.01-0.12, and the mixture is formulated in a known way with one or more additives into a pharmaceutical product. If instead of the macrolide antibiotic chloramphenicol or thiamphenicol is used, its preferable weight ratio in the above mixture is 0.01-7.0.

An exemplary composition contains the following ingredients:

| | |
|---|---|
| chloramphenicol | 0.5- 2.0 g |
| sulfadimidine | 2.0- 10.0 g |
| zinc oxide | 1.5- 8.3 g |
| camomile oil | 0.3- 3.28 g |
| azulene | 0.01- 0.12 g |
| cetyt-stearylalcohot | 0.0- 5.0 g |
| beeswax | 2.5- 5.5 g |
| ophthalmic vaseline | 60.0-85.0 g |
| lanalcolum | 1.58- 5.12 g |

Another preferable composition contains the following ingredients:

| | |
|---|---|
| primycin-sulfate | 0.1- 1.0 g |
| sulfamethoxazole | 2.0- 10.0 g |
| zinc oxide | 1.5- 8.3 g |
| cetyl-stearylalcohol | 0.0- 5.0 g |
| beeswax | 2.5- 5.5 g |
| lanalcolum | 1.58- 5.12 g |
| azulene | 0.01- 0.12 g |
| camomile oil | 0.3- 3.28 g |
| ophthalmic vaseline | 60.0- 85.0 g |

In the compositions of the invention azulene can be used also in the form of volatile oils containing azulene, e.g. in the form of blood-wort (achillea) oil.

As during the burning the tissues on the surface are damaged to different extents and the sensory nerve-terminations are exposed to a shock-like effect, the injury is extremely painful. The composition of the invention covers the wounded area and isolates it from its surroundings. The active ingredients penetrate into the injury leaving the healthy tissues intact. Oxygen or air which is absorbed by the pain sensitive receptors under the influence of the heat, and the irritating tissue ingredients formed during the burning are removed from the receptors, thereby their constant irritation ceases, thus pain is reduced. Therefore when using the compositions of the invention, pain alleviation only in extreme cases is necessary. The composition creates a "tissue friendly" environment, it does not obstruct the patient's movements and an epidermis is developed which is not yet hornified or pigmented. Following gradual adaptation to light pigmentation begins spontaneously and also hornification occurs. The surface of the skin will only show a slight difference when compared to its state before the injury, as a result no plastic surgery is needed.

In general after use for 20 days a slight allergic reaction might be expected, which means that expansive or total bums cannot be treated until complete healing has taken place. In such cases - as no intensive proliferation or epithelisation is needed- follow-up treatment is advisable using an attenuated version of the composition.

The composition suitable for follow-up treatment contains as active ingredient zinc oxide, camomile oil and azulene, preferably in the weight ratio of 3.2-5.0 : 0.5-3.2 : 0.01-0.12. This composition is prepared by formulating the above mixture in a known way with one or more additives into a pharmaceutical composition.

An examplary composition for follow-up treatment contains the following ingredients:

| | |
|---|---|
| zinc oxide | 3.2- 6.0 g |
| camomile oil | 0.1- 3.2 g |
| azulene | 0.01- 0.12 g |
| cetyl-stearylalcohol | 1.65- 3.0 g |
| beeswax | 3.2- 5.5 g |
| ophthalmic vaseline | 65.0- 90.0 g |
| lanalcolum | 3.0- 6.0 g |

The compositions of the invention are prepared from ingredients of pharmacopeal quality.

The compositions of the invention can be effectively used also for curing slowly healing wounds and external ulcers treated in the traditional way.

The invention is illustrated by the following examples.

### Example 1

Ointment for treating burns and external ulcers

| Ingredients: | |
|---|---|
| 1.28 g | of chloramphenicol, |
| 6.60 g | of sulfadimidine, |
| 3.20 g | of zinc oxide. |
| 2.50 g | of aetheroleum chamomillae, |
| 0.04 g | of azulene in the form of aetheroleum millefolii, |
| 1.65 g | of cetyl-stearylalcohol, |
| 3.18 g | of cera alba, |
| 78.30 g | of vaselinum album ophthalmicum, |
| 3.45 g | of lanalcolum |

A small amount of the mixture of beeswax, wool wax, cetyl-stearylalcohol and ophthalmic vaseline melted on a water bath and cooled under continuous stirring to 40 °C is homogenized with the mixture of chloramphemicol, sulfadimidine and zinc oxide. The homogeneous mass is mixed with the rest of the ingredients and it is left to cool for 12 hours, then it is homogenized once again. Thereafter the aetheroleum chamomillae (camomile oil) and the aetheroleum millefolii (blood-wort oil) containing the defined amount of azulene are added to the ointment.

The ointment thus obtained can be directly applied onto the wound but in the case of sensitive and painful wounds it is preferable to spread it onto a thin layer of gauze and then place it on the wound.

### Example 2

Ointment for the treatment of burns and external ulcers

The ointment is prepared as desribed in example 1 from the following ingredients:

| | |
|---|---|
| 0.20 g | of primycin-sulfate, |
| 3.50 g | of sulfamethoxazole, |
| 3.20 g | of zinc oxide, |
| 1.65 g | of cetyl-stearylalcohol, |
| 3.18 g | of cera alba, |
| 3.45 g | of lanalcolum, |
| 0.04 g | of azulene, |
| 0.60 g | of aetheroleum chamomillae, |
| 84.20 g | of vaselinum album ophthalmicum. |

### Biological tests

### Treatment of burns

Forty-six patients who suffered burnings were treated with the ointment described in the example 1. The age of the patients ranged between 11 months and 68 years. The size of the injuries varied between 5 cm² and 2000 cm², most of which were second or third degree burns. Scalding was the major cause of the wounds.

100 % of the cases healed without scars. The table below shows the healing times and the number of cases.

**Table**

| | Healing time | No. of cases |
|---|---|---|
| First degree burns | 2- 4 days | 6 |
| First-second degree burns | 3- 6 days | 22 |
| Second-third degree burns | 6-14 days | 18 |

### Treatment of external ulcers

Twenty-eight patients suffering from external ulcers mostly due to unsatisfactory blood circulation were treated. They had sweating, painful wounds, contaminated by necrotised tissue elements, of varying depths (between 5 and 20 mm), slightly swollen at the edges, of different sizes (between 4 cm² and 700 cm²) and having been open for several years. The presence of two wounds -one of them being venous, the other one arterial- on one limb being often the case.

After treatment with the ointment described in example 1 the pain ceased on the first day and sweating diminished. With changing the bandage every 24 hours the necrotised tissue elements were removed and the wound became cleaner. Over the following days the deep ulcers started to fill up and the epithelising process commenced at the edges. The continuous filling up of the wound did not lead to proliferation, the epidermis did not crease but stuck to the base of the skin. The composition ensured that the wound was free from bacteria and sterile, thus infection was undetectable. In all the cases, a significant recovery could be observed, compared to traditional cures.

## Claims

1. Composition for treating burns and external ulcers, which contains as active ingredient the mixture of one or more sulfonamides, a macrolide antibiotic or chloramphenicol or thiamphenicol, together with zinc oxide, camomile oil and azulene.

2. The composition according to claim 1, wherein the sulfonamide is sulfadimidine.

3. The composition according to claim 1, wherein the macrolide antibiotic is primycin-sulfate

4. The composition according to claim 1, which comprises containing the sulfonamide, the macrolide antibiotic, zinc oxide, camomile oil and azulene in a weight ratio of 0.1-15.0 : 0.1-1.0 : 0.1-25.0 : 0.3-5.0 : 0.01-1.0, especially 2.0-10.0 : 0.5-2.0 : 1.5-8.3 : 0.3-3.28 : 0.01-0.12.

5. The composition according to claim 1, which comprises containing a sulfonamide, chloramphenicol or thiamphenicol, zinc oxide, camomile oil and azulene in a weight ratio of 0.1-15.0 : 0.01-7.0 : 0.1-25.0 : 0.3-5.0 : 0.01-1.0, especially 2.0 -10.0 : 0.5-2.0 : 1.5-8.3 : 0.3-3.28 : 0.01-0.12.

6. The composition according to claim 1, wherein the azulene is a volatile oil containing azulene.

7. Process for the preparation of compositions for treating burns or external ulcers, which comprises mixing one or more sulfonamides with a macrolide antibiotic or chloramphenicol or thiamphenicol and zinc oxide, camomile oil and azulene.

8. The process according to claim 7, which comprises formulating the mixture with one or more additives into a pharmaceutical product.

## Patentansprüche

1. Zusammensetzung zum Behandeln von Verbrennungen und äußerlichen Geschwüren, die als Wirkstoff die Mischung aus einem oder mehreren Sulfonamiden, einem Makrolid-Antibiotikum oder Chloramphenicol oder Thiamphenicol, zusammen mit Zinkoxid, Kamillenöl und Azulen, enthält.

2. Zusammensetzung nach Anspruch 1, wobei das Sulfonamid Sulfadimidin ist.

3. Zusammensetzung nach Anspruch 1, wobei das Makrolid-Antibiotikum Primycinsulfat ist.

4. Zusammensetzung nach Anspruch 1, die das Sulfonamid, das Makrolid-Antibiotikum, Zinkoxid, Kamillenöl und Azulen in einem Gewichtsverhältnis von 0,1-15,0:0,1-1,0:0,1-25,0:0,3-5,0:0,01-1,0, insbesondere von 2,0-10,0:0,5-2,0:1,5-8,3:0,3-3,28:0,01-0,12, enthält.

5. Zusammensetzung nach Anspruch 1, die ein Sulfonamid, Chloramphenicol oder Thiamphenicol, Zinkoxid, Kamillenöl und Azulen in einem Gewichtsverhältnis von 0,1-15,0:0,01-7,0:0,1-25,0:0,3-5,0:0,01-1,0, insbesondere von 2,0-10,0:0,5-2,0:1,5-8,3:0,3-3,28:0.01-0,12, enthält.

6. Zusammensetzung nach Anspruch 1, wobei das Azulen ein azulenhaltiges flüchtiges Öl ist.

7. Verfahren zur Herstellung von Zusammensetzungen zum Behandeln von Verbrennungen oder äußerlichen Geschwüren, das ein Mischen eines oder mehrerer Sulfonamide mit einem Makrolid-Antibiotikum oder Chloramphenicol oder Thiamphenicol und Zinkoxid, Kamillenöl und Azulen umfasst.

8. Verfahren nach Anspruch 7, das ein Formulieren der Mischung mit einem oder mehreren Zusätzen in ein pharmazeutisches Produkt umfasst.

## Revendications

1. Composition de traitement de brûlures et de plaies externes qui contient, en tant qu'ingrédient actif, le mélange d'un ou de plusieurs sulfonamides, d'un antibiotique de type macrolides ou de chloramphénicol ou de thiamphénicol, avec de l'oxyde de zinc, de l'huile de camomille et de l'azulène.

2. Composition selon la revendication 1, dans laquelle le sulfonamide est la sulfadimidine.

3. Composition selon la revendication 1, dans laquelle l'antibiotique de type macrolides est le sulfate de pimycine.

4. Composition selon la revendication 1 qui comprend le sulfonamide, l'antibiotique de type macrolides, l'oxyde de zinc, l'huile de camomille et l'azulène dans un rapport en poids de 0,1-15,0 : 0,1-1,0 : 0,1-25,0 : 0,3-5,0 : 0,01-1,0, en particulier de 2,0-10,0 : 0,5-2,0 : 1,5-8,3 : 0,3-3,28 : 0,01-0,12.

5. Composition selon la revendication 1 qui comprend le sulfonamide, le chloramphénicol ou le thiamphénicol, l'oxyde de zinc, l'huile de camomille et l'azulène dans un rapport en poids de 0,1-15,0 : 0,01-7,0 : 0,1-25,0 : 0,3-5,0 : 0,01-1,0, en particulier de 2,0-10,0 : 0,5-2,0 : 1,5-8,3 : 0,3-3,28 : 0,01-0,12.

6. Composition selon la revendication 1, dans laquelle l'azulène est une huile volatile contenant de l'azulène.

7. Procédé de préparation de compositions de traitement de brûlures ou de plaies extérieures, qui comprend le mélange d'un ou de plusieurs sulfonamides avec un antibiotique de type macrolides ou du chloramphénicol ou du thiamphénicol, et de l'oxyde de zinc, de l'huile de camomille et de l'azulène.

8. Procédé selon la revendication 7, qui comprend la formulation du mélange avec un ou plusieurs additifs pour donner un produit pharmaceutique.
